# EUROPÄISCHE PATENTSCHRIFT

(11) **EP 1 030 626 B1**
(45) Veröffentlichungstag und Bekanntmachung des Hinweises auf die Patenterteilung: **05.02.2003**
(21) Anmeldenummer: 98959856.0
(22) Anmeldetag: 06.11.1998
(51) Int. Cl.: A61F 2/30, A61F 2/32, A61F 2/38

(54) **GLEITPARTNER FÜR KÜNSTLICHE GELENKIMPLANTATE**
SLIDING PARTNER ELEMENTS FOR ARTIFICIAL JOINT IMPLANTS
ELEMENTS COMPLEMENTAIRES GLISSANTS DESTINES A DES IMPLANTS D'ARTICULATION ARTIFICIELS

(30) Priorität: 13.11.1997 DE 19750121
(43) Veröffentlichungstag der Anmeldung: 30.08.2000
(73) Patentinhaber: ESKA Implants GmbH & Co., 23556 Lübeck (DE)
(72) Erfinder: GRUNDEI, Hans, D-23558 Lübeck (DE); THOMAS, Wolfram, I-00135 Rom (IT)
(74) Vertreter: Fuchs Mehler Weiss & Fritzsche
(86) Internationale Anmeldenummer: EP9807087
(87) Internationale Veröffentlichungsnummer: WO99025276

(56) Entgegenhaltungen:
- EP-A- 0 373 011
- EP-A- 0 827 726
- WO-A-96/19162
- DE-A- 4 336 932
- DE-A- 4 423 020
- GB-A- 1 322 680
- GB-A- 1 349 987
- GB-A- 1 527 498
- US-A- 4 676 799
- US-A- 4 840 631
- US-A- 5 462 362
- US-A- 5 683 466

## Beschreibung

Die vorliegende Erfindung betrifft eine flächenhafte Paarung von Gleitpartnern für künstliche Gelenkimplantate, bei denen ein Gelenkteil gegenüber einem anderen Gelenkteil eine Bewegung, die eine Rotations-, Abroll- oder Gleitbewegung oder eine Kombination aus diesen sein kann, ausführt.

Derartige künstliche Gelenkimplantate sind in erster Linie künstliche Hüftgelenke, Kniegelenke, Schultergelenke und Sprunggelenke. Generell läßt sich die Erfindung jedoch auf alle künstlichen Gelenke anwenden, bei denen zwei Gleitpartner aufeinandertreffen.

Generell ist man bemüht, die Reibung zwischen den sich relativ zueinander bewegenden Gelenkteilen möglichst gering zu halten. Ein positiver Nebeneffekt hiervon ist, daß der Partikelabrieb mit abnehmendem Reibungskoeffizienten deutlich geringer wird, was der Langzeitstabilität des Implantates zugute kommt.

Nach der Implantation der künstlichen Gelenke werden die Gelenkteile von natürlicher Gelenkflüssigkeit, der sogenannten Synovia, umspült. Bei passgenau gearbeiteten Gelenkteilen, beispielsweise Gelenkkugeln und Gelenkpfannen eines künstlichen Hüftgelenkes, kann es dazu kommen, daß der Flüssigkeitsfilm zwischen den sich gegeneinander bewegenden Gelenkteile abreißt und so keinerlei Schmierfunktion mehr ausüben kann. Hierdurch wird die Reibung zwischen den Gelenkteilen abrupt deutlich erhöht, wodurch die Funktionsfähigkeit des künstlichen Gelenkes beeinträchtigt wird und dem Abrieb von Partikeln der Gleitpartner Vorschub geleistet wird. Hierdurch können erhebliche Probleme im Hinblick auf den Langzeitverbleib des Implantates im Patientenkörper resultieren.

Im Stand der Technik gemäß der GB 1 527 498 ist bereits vorgeschlagen worden, dieses Problem zu lösen. So sind z.B. an der Grenzfläche zwischen dem Gleitpartner in einer künstlichen Beckenpfanne und dem künstlichen Hüftgelenkskopf Vertiefungen in Form von untereinander in Verbindung stehender Kanäle vorgesehen, in denen die Körperflüssigkeit für die Schmierung des Gelenkes sorgen soll. Ganz ähnliche Konstruktionen sind bekannt geworden aus der DE 43 36 932 C2 und DE 44 23 020 A1. Dadurch, daß die flüssigkeitsführenden Kanäle miteinander kommunizieren, kann es zu Problemen hinsichtlich des Trockenfallens einiger Kanäle an exponierten Stellen kommen. Diesem Trockenfallen folgt dann das Problem der ungleichmäßigen Schmierung des künstlichen Hüftgelenkes, wodurch dessen Funktionsfähigkeit in negativer Weise beeinträchtigt wird.

Aus der US 5,462,362 A sind abriebsfeste flächenhafte Paasungen von Gleitpartner gemäß dem Oberbegriff des Anspruchs 1 bekannt, die auch Verwendung finden sollen bei Gelenkimplantaten. In die Oberfläche der Gelenkteile sind dabei Vertiefungen eingelassen, die ein Reservoir für Körperflüssigkeit darstellen, welche einen Flüssigkeitsfilm zwischen den sich gegeneinander bewegenden Teilen ausbildet. Die Vertiefungen sind hier durchgängig als zylindrische oder Sacklöcher ausgebildet. Das Problem hierbei ist, daß eine abpuffernde Funktion der Körperflüssigkeit in diesem Zusammenhang nur unzureichend ausgeübt wird. Letztlich ist dies eine Folge der Druckverteilung aufgrund der zylindrischen Ausbildung der Vertiefung. Dadurch kann die Synovia ihre Funktion nicht zuverlässig ausüben.

Vor diesem Hintergrund ist es die Aufgabe der vorliegenden Erfindung, derartig beschriebene flächenhafte Paarungen von Gleitpartnern für künstliche Gelenkimplantate so weiterzubilden, daß der Flüssigkeitsfilm der Synovia im Spalt zwischen den Gelenkteilen mit hoher Wahrscheinlichkeit nicht abreißt, so daß die Synovia ihre Funktion als Gelenkschmierflüssigkeit zuverlässig ausüben kann.

Gelöst wird diese Aufgabe durch die flächenhaften Paarungen von Gleitpartnern gemäß dem Anspruch 1. Vorteilhafte Weiterbildungen sind in den Unteransprüchen angegeben.

Demgemäß wird vorgeschlagen, daß neben dem Spalt für die Synovia zwischen den Gleitpartnern die Oberfläche zumindest eines Gleitpartners regelmäßig angeordnete Vertiefungen aufweist, die nicht zusammenhängend, also ohne Verbindungskanäle untereinander, ausgeführt wird. Die Vertiefungen in der Oberfläche eines Gleitpartners üben eine Sammelfunktion für die Synovia aus, die ihrerseits einen puffernden Effekt hin zum anderen Gleitpartner ausübt. Die flüssigkeitssammelnde Funktion der Vertiefungen sorgt dafür, daß es nicht zu einem mehr oder weniger abrupt einsetzenden Abriß des Flüssigkeitsfilmes zwischen den Gleitpartnern kommen kann. Stets ist dafür Sorge getragen, daß die Gleitpartner nicht direkt aufeinander abrollen oder aufeinander gleiten, sondern stets ein Film von Synovia zwischen ihnen die Reibung reduziert. Weniger Abriebspartikel sind die unmittelbare Folge, wodurch die Langzeitstabilität des Implantates noch verbessert wird.

Die Vertiefungen sind im Querschnitt kalottenförmig ausgebildet. Hierdurch bieten sie einerseits ein genügend großes Reservoir für die Synovia und andererseits die Möglichkeit einer guten Umspülung und Austausch der in der Vertiefungen gespeicherten Synovia gegen nachströmende Synovia.

Gemäß einer vorteilhaften Weiterbildung ist vorgesehen, daß die Vertiefungen bis zu 80% der Oberfläche des wenigstens einen Gleitpartners einnehmen.

Hierdurch wird ein relativ großes Volumen an Synovia quasi in einem Reservoir vorgehalten, um so den puffernden Effekt zu erzielen.

Die Vertiefungen können eine hexagonale Öffnung aufweisen, also in Projektion ein wabenförmiges Erscheinungsbild aufweisen. Alternativ hierzu können die Öffnungen auch rund ausgebildet sein. Die Auswahl der einen oder anderen Möglichkeit hängt unter anderem ab von der Gesamtfläche, den zu erwartenden Flächendrücken und von der Anordnung auf der Oberfläche des betreffenden Gelenkteiles.

Gemäß einer bevorzugten Ausführungsform sind die Vertiefungen in der Oberfläche eines Gleitpartners desjenigen Gelenkteils verteilt angeordnet, welches sich gegenüber dem anderen, ortsfesten Gelenkteil bewegt. Mit anderen Worten sind die Vertiefungen in diesem Falle in der Oberfläche des Gelenkteils ausgebildet, welches gegenüber dem anderen Gelenkteil die Abroll-, Roll-, Rotations- oder Gleitbewegung ausführt.

Im Falle eines künstlichen Hüftgelenkes wären die Vertiefungen bei dieser Ausführungsform also in der Gelenkkugel vorgesehen, wohingegen der Gleitpartner im Acetabulum glattflächig ausgebildet sein kann.

Die Paarungen von Gleitpartnern sind vorzugsweise ausgewählt aus der Gruppe der Paarungen Metall-Metall, Keramik-Keramik, Keramik-Metall, Polyethylen-Metall oder Polyurethan/Keramikcompound-Metall.

Die Erfindung wird anhand einiger Ausführungsbeispiele gemäß der Zeichnungsfiguren näher erläutert.

Hierbei zeigt:
- Figur 1a: eine Schnittansicht durch eine künstliche Gelenkpfanne mit Inlet, in welchem sich eine Gelenkkugel abwälzt,
- Figur 1b: die Ansicht einer künstlichen Gelenkkugel,
- Figur 1c: die Schnittansicht der Gelenkkugel aus Figur 1b,
- Figur 2a: eine Seitenansicht des Femurkondylenteils eines künstlichen Kniegelenkes,
- Figur 2b: die Ansicht auf das Femurkondylenteil aus Figur 2a von dorsal,
- Figur 2c: die Aufsicht auf das Femurkondylenteil gemäß den Figuren 2a und 2b,
- Figur 3a: die Ansicht auf ein Tibiaplateauteil eines künstlichen Kniegelenkes von unten,
- Figur 3b: die Schnittansicht durch das Tibiaplateau, und
- Figur 3c: die Aufsicht auf die Gleitbahnen des Tibiaplateaus gemäß den Figuren 3a und 3b.

Nachfolgend sind funktionell gleiche Teile mit denselben Bezugszeichen versehen.

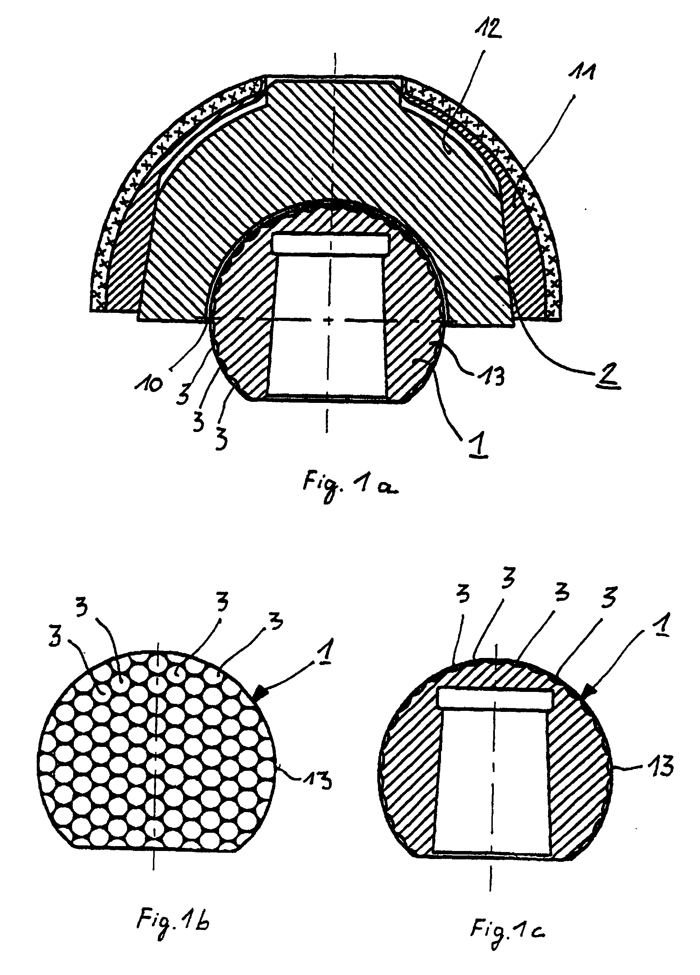

Einen ersten Überblick verschafft Figur 1a.

Darin gezeigt ist eine künstliche Gelenkpfanne, bestehend aus einem Metallsockel 11, der in das ausgefräste Acetabulum im natürlichen Beckenknochen gesetzt wird und dort fixiert wird. Als Gleitpartner des ortsfesten Gelenkteiles 2 dient das Inlet 12, welches in den Metallsockel 11 eingepaßt ist. In eine kalottenförmige Ausnehmung im Inlet 12 greift eine künstliche Gelenkkugel 13 als bewegliches Gelenkteil 1. Zwischen der Gelenkkugel 13 und der kalottenförmigen Ausnehmumg im Inlet 2 ist ein Spalt 10 ausgebildet, der nach der Implantation mit Synovia gefüllt ist. Damit dieser Flüssigkeitsfilm nicht abreißt, womit das Gelenk trockenlaufen würde, sind auf der Oberfläche der Gelenkkugel 13 regelmäßig angeordnete Vertiefungen 3 vorgesehen, die in die Oberfläche eingelassen sind. Die Vertiefungen füllen sich nach der Implantation mit Synovia und bilden somit ein Reservoir für Synovia, womit sichergestellt ist, daß der Flüssigkeitsfilm im Spalt 10 nicht zum Abreißen kommt.

Welch große Bereiche die Vertiefungen 3 auf der Oberfläche der Gelenkkugel 13 einnehmen, wird sehr anschaulich aus Figur 1b, wo eine Ansicht der Gelenkkugel 13 gezeigt ist. Bis zu 90% der Oberfläche der Gelenkkugel 13 wird von den Vertiefungen 3 eingenommen. Die verbleibenden Oberflächenteile bilden die tatsächlichen Reibflächen mit dem Inlet 12. Im dargestellten Ausführungsbeispiel sind die Öffnungen der Vertiefungen rund. Alternativ können diese hexagonal ausgebildet sein, wobei sich dann allerdings die Oberflächenanteile, welche tatsächlich in der Kalotte im Inlet 12 abrollen, verändern.

Figur 1c zeigt die Gelenkkugel 13 nochmals im Schnittbild und dient zur Abrundung des Gesamtbildes.

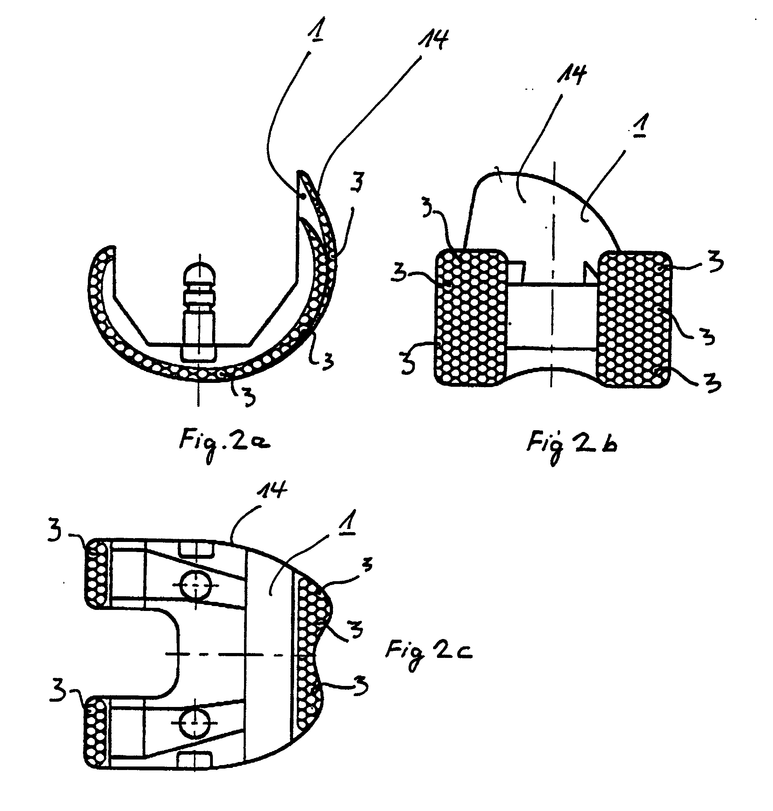

In der Figur 2a ist eine Seitenansicht eines Femurkondylenteiles 14 eines ersten Gelenkteiles 1 eines künstlichen Kniegelenkes gezeigt. Die Kondylen sind vorliegend überall mit den Vertiefungen 3 versehen. Insbesondere sind die hinteren Kondylen, wie aus Figur 2b ersichtlich, mit den Vertiefungen versehen, da diese den maßgeblichen Anteil an der Roll- und Gleitbewegung auf dem Gegenstück eines (nicht dargestellten) Tibiateils des Kniegelenkes innehaben. Figur 2c ist die Ansicht auf das Femurkondylenteil 14 von oben und rundet das Bild ab.

Figur 3a zeigt ein Tibiaplateauteil 15 als zweites (ortsfestes) Gelenkteil 2 eines künstlichen Kniegelenkes von unten her gesehen. Genauer gesagt bildet das Tibiaplateau 15 den Gleitpartner des zweiten (orstfesten) Gelenkteiles 2 des nicht dargestellten Tibiastielteils eines künstlichen Kniegelenks.

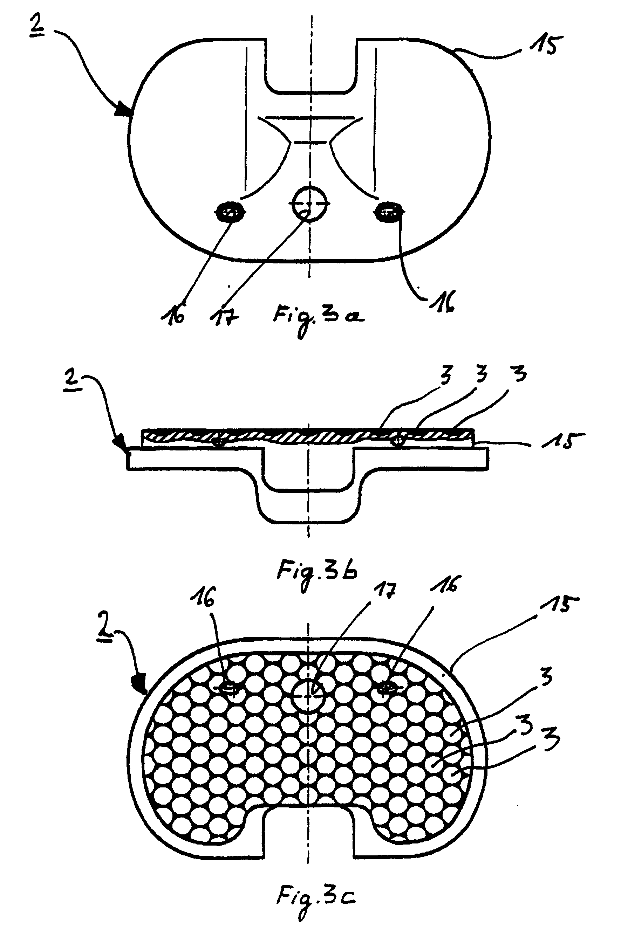

Die dargestellte Ausführung weist eine Durchbrechung 17 auf, durch welche hindurch ein Rotationszapfen greifen kann. Öffnungen 16 dienen zum Durchtritt der Synovia auch in den Zwischenraum zwischen dem (nicht dargestellten) Tibiastielteil und dem Tibiaplateau 15. Dies ist aus Figur 3b ersichtlich. Mit anderen Worten sorgen die Öffnungen 16 für Hindurchtritt von Synovia hin zum Tibiastielteil (nicht dargestellt), um der schwimmenden Lagerung des Plateaus 15 am Tibiastielteil Vorschub zu leisten. Hierfür nämlich greift der (nicht dargestellte) Rotationszapfen durch die Durchbrechung 17, so daß sich das Plateau 15 in gewissem Maße gegenüber dem Tibiastielteil bewegen kann. Die dem Femurkondylenteil mit beispielsweise glatten Kondylen zugewandte Oberseite des Tibiaplateaus 15 ist mit den Vertiefungen 3 versehen. Auch hier dienen die Vertiefungen als Reservoir für Synovia, so daß stets für einen ausreichenden Flüssigkeitsfilm zwischen dem Tibiaplateau mit seinen Gleitbahnen und dem Femurkondylenteil gesorgt ist.

Die große Dichte der Vertiefungen 3 im Tibiaplateau 15 auf dessen Oberfläche ist in Figur 3c veranschaulicht. Die große Anzahl der Vertiefungen 3, die bis zu 90% der gesamten Oberfläche des Gleitpartners einnehmen können, sorgt für einen entsprechend großen Vorrat an Synovia in den Vertiefungen und damit für eine hinreichend große Bevorratung an Synovia. Die Ausbildung der Vertiefungen 3 in diesem Ausführungsbeispiel entspricht jener bei dem künstlichen Hüftgelenk gemäß den Figuren 1a bis 1c.

## Patentansprüche

1. Flächenhafte Paarungen von Gleitpartnern für künstliche Gelenkimplantate, bei denen ein Gelenkteil (1) gegenüber einem anderen Gelenkteil (2) eine Rotations-, Abroll- oder Gleitbewegung bzw. eine Kombination aus diesen ausführt, wobei zwischen den Gleitpartnern ein Spalt (10) für einen natürlichen Flüssigkeitsfilm, bespielsweise einem Film aus Synovia, ausgebildet ist, und die Oberfläche zumindest eines Gleitpartners regelmäßig angeordnete Vertiefungen (3) ohne Verbindungskanäle untereinander aufweist, **dadurch gekennzeichnet, dass** die Vertiefungen (3) im Querschnitt kalottenförmig ausgebildet sind.

2. Gleitpartner nach Anspruch 1, bei denen die Vertiefungen (3) bis zu 90% der Oberfläche des wenigstens einen Gleitpartners einnehmen.

3. Gleitpartner nach Anspruch 1 oder 2, bei denen die Vertiefungen (3) eine hexagonale Öffnung aufweisen.

4. Gleitpartner nach Anspruch 1 oder 2, bei denen die Vertiefungen (3) eine runde Öffnung aufweisen.

5. Gleitpartner nach einem der Ansprüche 1 bis 4, bei denen die Vertiefungen (3) auf der gegenüber der Oberfläche des ortsfesten Gelenkteiles (2) beweglichen Oberfläche eines Gleitpartners verteilt angeordnet sind.

6. Paarungen von Gleitpartnern nach einem der Ansprüche 1 bis 5, wobei sie aus der Gruppe der Paarungen Metall-Metall, Keramik-Keramik, Keramik-Metall, Polyethylen-Metall oder Polyurethan/Keramikcompound-Metall ausgewählt ist.

## Claims

1. Two-dimensional pairings of sliding partners for artificial joint implants, in which a joint part (1) executes a rotary, rolling or sliding movement or a combination of these with respect to a further joint part (2), wherein a gap (10) for a natural fluid film, for example a film of synovial fluid, is formed between the sliding partners, and the surface of at least one sliding partner has regularly arranged depressions (3) without mutual connecting channels, **characterised in that** the depressions (3) are designed to have dome-shaped cross-section.

2. Sliding partner according to claim 1, in which the depressions (3) occupy up to 90% of the surface of the at least one sliding partner.

3. Sliding partner according to claim 1 or 2, in which the depressions (3) have a hexagonal opening.

4. Sliding partner according to claim 1 or 2, in which the depressions (3) have a round opening.

5. Sliding partner according to one of claims 1 to 4, in which the depressions (3) are arranged distributed on the moveable surface of a sliding partner opposite the surface of the fixed joint part (2).

6. Pairings of sliding partners according to one of claims 1 to 5, wherein it is selected from the group of pairings metal-metal, ceramic-ceramic, ceramic-metal, polyethylene-metal or polyurethane/ceramic compound-metal.

## Revendications

1. Appariements plans de partenaires de glissement pour des implants d'articulation artificiels, sur lesquels une partie d'articulation (1) exécute par rapport à une autre partie d'articulation (2) un mouvement de rotation, de déroulement ou de glissement ou une combinaison de ces mouvements, une fente (10) pour une pellicule de liquide naturel, par exemple une pellicule à base de synovie, étant ménagée entre les partenaires de glissement, et la surface d'au moins un partenaire de glissement présente des cavités (3) disposées de façon régulière sans canaux de liaison entre elles, **caractérisés en ce que** les cavités (3) sont conçues en forme de calottes vues en section.

2. Partenaires de glissement selon la revendication 1, avec lesquels les cavités (3) occupent jusqu'à 90 % de la surface d'au moins un partenaire de glissement.

3. Partenaires de glissement selon la revendication 1 ou 2, avec lesquels les cavités (3) présentent une ouverture hexagonale.

4. Partenaires de glissement selon la revendication 1 ou 2, avec lesquels les cavités (3) présentent une ouverture ronde.

5. Partenaires de glissement selon l'une quelconque des revendications 1 à 4, avec lesquels les cavités (3) sont disposées de façon répartie à la surface d'un partenaire de glissement, mobile par rapport à la surface de la partie d'articulation (2) fixe.

6. Appariements de partenaires de glissement selon l'une quelconque des revendications 1 à 5, qui sont choisis dans le groupe des appariements métal-métal, céramique-céramique, céramique-métal, polyéthylène-métal ou polyuréthane/ céramique mixte-métal.
